# EUROPEAN PATENT APPLICATION

(11) **EP 4 656 245 A1**
(43) Date of publication of application: **03.12.2025**
(21) Application number: 24178299.4
(22) Date of filing: 27.05.2024
(51) Int. Cl.: A61P 31/04, C07D 233/64, C07K 7/06, A61K 31/4164

(54) **HISTIDINE-BASED UNNATURAL AMINO ACID, ITS PRODUCTION AND USE**

(71) Applicant: PHILIPPS-UNIVERSITÄT MARBURG, 35037 Marburg (DE)
(72) Inventor: Graumann, Peter, 35043 Marburg (DE); Kilb, Alexandra, 35032 Marburg (DE); Klee, Dennis, 35032 Marburg (DE); Trinh, Van Tuan, 35032 Marburg (DE); Geyer, Armin, 35043 Marburg (DE); Vázquez, Olalla, 35043 Marburg (DE)
(74) Representative: Durm Patentanwälte PartG mbB

(57) **Abstract**

Described is a compound for substituting amino acids of proteins or peptides, wherein the compound has the formula (1) 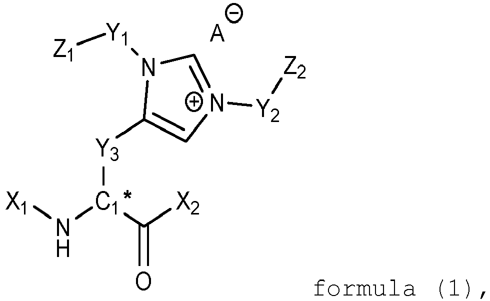 wherein the stereocenter C₁* represents C*H* that either results in the D- or L-form of the compound of formula (1); A⁻ is a negatively charged ion compensating the positive charge; X₁ is H or an amine protecting group; X₂ is OH, an ester residue or an amide residue or an activated carboxylic acid derivate to be used for the formation of an amide bond; Y₁, Y₂ and Y₃ are independently from each other a C₁-C₆ alkyl, and Z₁ and Z₂ are independently from each other a nucleobase, a C1 to C16 alkyl group, an aromatic residue or a heteroaromatic residue, wherein the aromatic residue or the heteroaromatic residue contains one 5, 6, or 7-membered ring or two condensed 5, 6, or 7-membered rings sharing two carbon atoms, wherein the aromatic residue or the heteroaromatic residue can be substituted by a linear or branched C1 to C16 alkyl group and/or a further aromatic group. Furthermore, a method for preparing this compound and proteins and peptides containing this compound are disclosed.

## Description

The present invention relates to a histidine-based unnatural amino acid, a method for its preparation and its use.

The invention also provides novel antimicrobial peptides containing histidine-based unnatural amino acids according to the present invention. Peptide-based antibiotics are used to treat bacterial infections. A major advantage over antibiotics based on organic molecules are metabolic degradation products, which are toxicologically more tolerable for the organism.

Bacteria can be categorised as Gram-positive and Gram-negative, which differ in the structure of their cell membrane. This can result in different pharmacological efficacies of an antibiotic against a certain class of bacteria. In particular, strains that have already developed resistance to common antibiotics (multi-resistant bacteria, ESKAPE) must be treated with new antibiotics. Combinations of several antibiotics are often administered to increase the sensitivity of the bacteria. Consequently, a large selection of antibiotics that are novel and widely applicable is constantly needed.

Antimicrobial peptides used to date can consist structurally of long peptides, as they are based on naturally occurring peptides. Their often non-linear and complex structure means that they can only be produced cost-effectively using recombinant production systems, which are associated with high development costs. Their size can lead to immune reactions when applied to organisms and they have significantly poorer biological availability.

Most antimicrobial peptides exert their effect via the nonspecific mechanism of cell lysis and can therefore cause side effects such as cell toxicity and haemolysis. A sufficient difference between the effective concentration and the concentration at which side effects occur must be ensured in order to guarantee a realisable and contractual treatment of infection. Furthermore, the method of administration of the peptide poses a further problem, as peptides are proteolytically degraded in serum.

The current peptide-based antibiotics consist of long and complex peptides. These are produced purely by biotechnology. Here are some examples of commercially available antimicrobial peptides:
- Vancomycin: use for severe infections following bacterial resistance;
- Gramicidin S: cyclic decapeptide, use as an external agent;
- Nisin: complex peptide, selective activity against Gram-positive strains, and
- Polymyxin B: cyclic peptide, selective activity against Gram-negative strains.

The technical problem underlying the present invention is to provide a compound for modifying proteins and peptides to fulfil critical requirements for use as an antibiotic and that can be used for the therapeutic treatment of infections with multi-resistant bacteria.

This is achieved by the subject-matter defined in the independent claims. Preferred embodiments are defined in the dependent claims.

According to the present invention, there is provided a compound for substituting amino acids of proteins or peptides, wherein the compound has the formula (1) 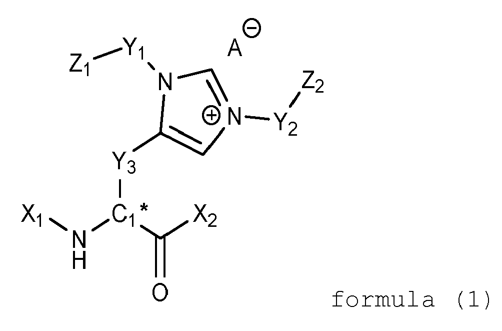 wherein
the stereocenter C₁* represents CH that either results in the D- or L-form of the compound of formula (1);
A⁻ is a negatively charged ion compensating the positive charge;
X₁ is H or an amine-protecting group;
X₂ is OH, an ester residue or an amide residue, or an activated carboxylic acid derivate, which are compounds that can be converted to carboxylic acids via acidic or basic hydrolysis, to be used for the formation of an amide bond;
Y₂, Y₂ and Y₃ are independently from each other a C₁-C₆ alkyl, in particular CH₂, and
Z₁ and Z₂ are independently from each other a nucleobase, which is a nitrogen-containing biological compound that form nucleosides, which, in turn, are components of nucleotides, a C1 to C16 alkyl group, an aromatic residue or an heteroaromatic residue, wherein the aromatic residue or the heteroaromatic residue contains one 5, 6, or 7-membered ring or two condensed 6, or 7-membered rings sharing two carbon atoms, wherein the aromatic residue or the heteroaromatic residue can be substituted by a linear or branched C1 to C 16 alkyl group, for example tert. butyl, and/or a further aromatic group, for example phenyl.

In one embodiment, A⁻ is a conjugate base selected from the group consisting of formic acid (COO⁻), acetic acid (CH₃COO⁻), trifluoroacetic acid (CF₃COO⁻) or halogen anions, in particular Br⁻, I⁻ and especially Cl⁻.

In a further embodiment, the amine-protecting group is selected from the group consisting of tert-butyloxycarbonyl (Boc-), allyloxycarbonyl (Alloc-), and especially the fluorenyl methoxycarbonyl protecting group (Fmoc-).

In another embodiment, the activated carboxylic acid derivate is selected from the group consisting of halogenides, in particular chloride (-Cl), *N*-hydroxysuccinimide ester (-NHS), hydroxybenzotriazole (-OBt) ester and *p*-nitrophenyl ester.

In one embodiment, Y₁, Y₂ and Y₃ can be defined as follows: Y₁ = Y₂ = Y₃, Y₁ ≠ Y₂ = Y₃, Y₁ = Y₂ ≠ Y₃, Y₂ = Y₃ ≠ Y₂ or Y₂ ≠ Y₂ ≠ Y₃.

Furthermore, in another embodiment Z₁ and Z₂ can or cannot be identical, i.e., Z₁ = Z₂. In particular, Z₁ and Z₂ are identical, and both are naphthyl, i.e., Z₁ = Z₂ = naphthyl.

As pointed out above, Z₁ and Z₂ can represent an aromatic or heteroaromatic residue containing of one or two fused 6-membered rings. Examples are given in the following:
a) 6-membered ring variants for Z₂: 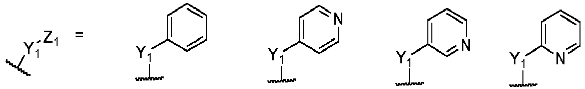 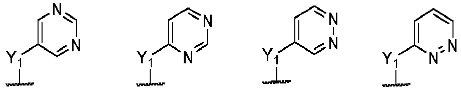 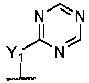 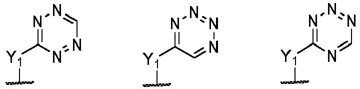
a) Two fused 6-membered ring variants for Z₁:

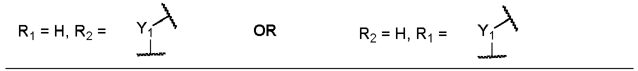

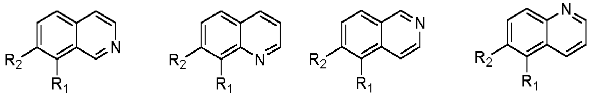

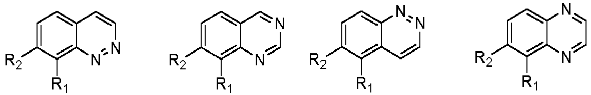

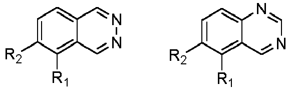

Heteroaromatic ring structures for Z₁ and Z₂ include also nucleobase variants

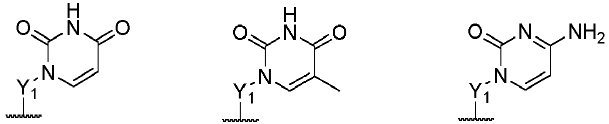

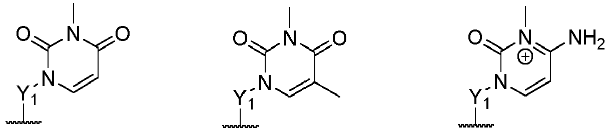

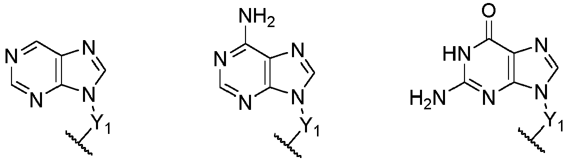

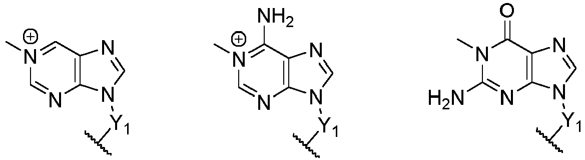

The wavy line as used in the present invention depicts the direct single bond to the nitrogen of the imidazole of formula (1) .

In particular, the compound according to the present invention can have the following selection of substituents:
Y₁ = Y₂ = Y₃ = CH₂; Z₁ = Z₂ = naphthyl; X₂ = OH, X₁ is Fmoc, and A⁻ is Cl⁻; this compound is designated in the following as "Hdn";
Y₁ = Y₂ = Y₃ = CH₂; Z₁ = Z₂ = methyl; X₂ = OH, X₁ is Fmoc, and A⁻ is Cl⁻; this compound is designated in the following as "Hdm";
Y₁ = Y₂ = Y₃ = CH₂; Z₁ = Z₂ = p-tert. butyl phenyl, X₂ = OH, X₁ is Fmoc, and A- is Cl-; this compound is designated in the following as "Hdt", and
Y₁ = Y₂ = Y₃ = CH₂; Z₁ = Z₂ = biphenyl; X₂ = OH, X₁ is Fmoc, and A⁻ is Cl⁻; this compound is designated in the following as "Hdb".

The present invention also provides a method for preparing in particular the compound of formula (1) as described above in detail, comprising the following steps:
esterification of Fmoc-His(Trt)-OH;
proton-catalyzed trityl (Trt) group deprotection;
direct dialkylation of the imidazole moiety, and
hydrolysis of the ester.

For example, the synthesis of the Fmoc-Hdn-OH building block or other compounds following the formula (1) can be based on a 4-step synthesis starting with the esterification of the commercially available Fmoc-His(Trt)-OH for example in MeOH and the presence of *N*-(3-dimethylaminopropyl)-*N*'-ethylcarbodiimide hydrochloride (EDCI) and 1-hydroxybenzotriazole (HOBt). The subsequent proton-catalyzed trityl (Trt) group deprotection (using for example AcOH in MeOH at 65°C for 1h) of this product can be followed up by a direct dialkylation of the imidazole moiety by 2-(bromomethyl) naphthalene or the corresponding bromo derivate of the groups Y₁-Z₁ (and Y₂-Z₂) with sodium bicarbonate (in for example MeCN at 65°C for 21h). After the final hydrolysis of the methyl ester (for example in 2M HCl in dioxane at 100°C for 16h), Fmoc-Hdn-OH or the other products of following the formula (1) can be obtained as hydrochloride salt in a yield of 98% over four steps

Further, the present invention also provides proteins or peptides, in which at least one amino acid of the protein or peptide is substituted by the compound of formula (1) as defined above.

The difference between a peptide and a protein is the length. According to the present invention, peptides having more than 50 amino acids are proteins.

In one embodiment, the peptide is an antimicrobial peptide. Antimicrobial peptides (AMPs)are found in all areas of life. Over 1200 peptides with an antimicrobial effect have been described to date. They serve as a defense against infection with microorganisms. Their effectiveness extends to Gram-negative and Gram-positive bacteria, enveloped viruses, fungi and tumor cells.

Antimicrobial peptides can be between 2 and 50 amino acids in length. They are categorized into different types according to their amino acid sequence: the loop-shaped peptides, the linear α-helical peptides, the extended peptides with an accumulation of one amino acid and the β-folded peptides with 2 or 3 disulphide bridges. They can contain two or more positively charged amino acids such as lysine, arginine and, in an acidic environment, histidine. They can also contain more than 50 % hydrophobic amino acids. The AMP can also be amidated at the C-terminus. Furthermore, also envisaged are cyclized version of the above-described AMP, for example head-to-tail, head-to-side chain, and side chain-to-side chain cyclization.

One example of an antimicrobial peptide is H₂N-RRWWRF-CONH₂(SEQ ID. no. 1), hereinafter referred to as "Com2".

All amino acids in the described proteins and peptides are interchangeable to their corresponding D- or L-configured counterparts.

Furthermore, all amino acids in the above-mentioned proteins and peptides are interchangeable to their homologues that differ solely by the length of the alpha-alkyl side chain in a range of ± C₁-C₃. In the following some examples are listed:

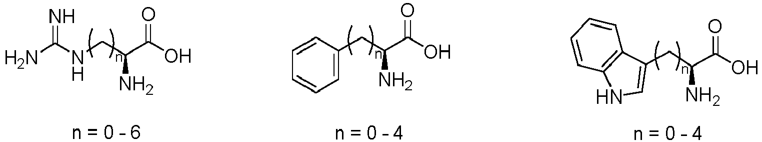

In one embodiment, the N-terminus of the protein or peptide, in which at least one amino acid is replaced by the compound of formula (1) is modified. That is, the N-terminus of the protein or peptide, in particular the antimicrobial peptide, and more particularly the antimicrobial peptide H₂N-RRWWRF-CONH₂ (SEQ ID no. 1) can be modified. This can be for example a 4-acetamidobenzoic acid-modified N-terminus. Such a modified N-terminus is hereby interchangeable with the free amine, as well as any N-terminal amide group that grants i) increased proteolytic stability and ii) increased hydrophobicity compared to the unmodified amine. In the following exemplified N-terminal chemical modifications are provided:

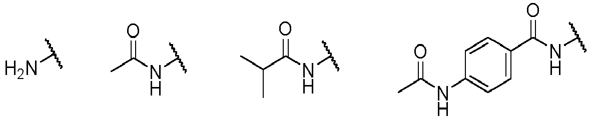

The C-terminal amide is hereby interchangeable with any C-terminal group that guarantees at least equal or i) increased proteolytic stability and ii) increased hydrophobicity compared to the unmodified free acid. In the following exemplified C-terminal chemical modifications are provided:

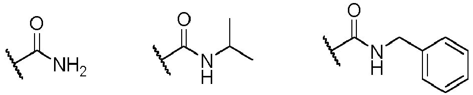

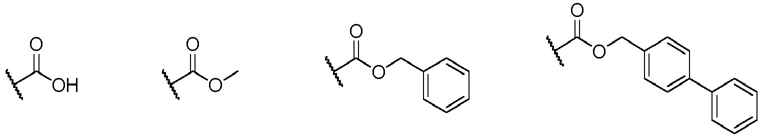

Additionally, to the examples provided above, single cyclization of i) head-to-tail, ii) tail-to-side chain, iii) head-to-side chain and iv) side chain-to-side chain type is also fulfilling these criteria.

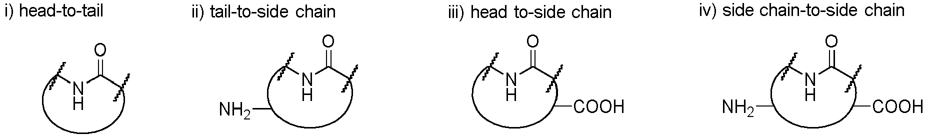

As pointed out above, at least one amino acid of the above-described proteins and peptides is substituted/replaced by the compound of formula (1) according to the present invention. In one embodiment, one to six amino acids of the protein or peptide are substituted by the compound of formula (1). It is possible, that the substitution occurs randomly or that at least two substitutions by the compound of formula up to all substitution are contiguous.

In the following table, various amino acid sequences are indicated as example of the above substitution, wherein the letter "B" stands for the histidine-based unnatural amino acid as defined in the above formula (1), in particular "B" stands for Hdn, Hdm, Hdt, and Hdb:

| Amino acid sequence | Seq Id no. | Abbreviation |
|---|---|---|
| RRWWRB | 2 | AMP2 |
| RRWWBF | 3 | AMP3 |
| RRWBRF | 4 | AMP4 |
| RRBWRF | 5 | AMP5 |
| RBWWRF | 6 | AMP6 |
| BRWWRF | 7 | AMP7 |
| RRWWBB | 8 | AMP8 |
| RRWBRB | 9 | AMP9 |
| RRBWRB | 10 | AMP10 |
| RBWWRB | 11 | AMP11 |
| BRWWRB | 12 | AMP12 |
| RRWBBF | 13 | AMP13 |
| RRBWBF | 14 | AMP14 |
| RBWWBF | 15 | AMP15 |
| BRWWBF | 16 | AMP16 |
| RRBBRF | 17 | AMP17 |
| RBWBRF | 18 | AMP18 |
| BRWBRF | 19 | AMP19 |
| RBBWRF | 20 | AMP20 |
| BRBWRF | 21 | AMP21 |
| BBWWRF | 22 | AMP22 |

It is clarified that the sequence listing only provides information about the amino acid sequence, but not the N- and C-terminal modifications. However, the present invention also envisages amino acid sequences having a C- and/or N-terminal modification. As an example: Com2 and P3 have the same sequence ID no. 1 but are different in their terminal modifications.

In one embodiment, the peptide according to the present invention has the formula RR-Hdn-Hdn-RF, which can be modified at the N-terminus by Aba resulting in Aba-RR-Hdn-Hdn-RF-CONH₂ (SEQ ID. No. 17), hereinafter referred to as "P14") wherein Hdn is the compound of formula (1) specifically defined above, and Aba represents a 4-acetamido benzoic acid residue.

The present invention also relates to the use of the compound of formula (1) as defined above in detail for replacing at least one amino acid of a protein or a peptide. The details of this use can be taken from the above description of the substitution of amino acids of proteins and peptide by the compound according to the present invention.

The above referred peptide P14 has been shown to fulfil critical requirements for use as an antibiotic and can be used for the therapeutic treatment of infections with multi-resistant bacteria.

Peptide P14 has a linear sequence of only 6 amino acids and an N-terminal modification. It can therefore be synthesised using solid-phase peptide synthesis (SPPS).

Like conventional antibiotics, the peptides and proteins according to the present invention, in particular peptide P14, can be dissolved in aqueous solvents such as physiological buffer or organic solvents such as DMSO. For use, the dissolved peptide can be applied to bacteria (in aqueous culture medium, on acute infection sites) to inhibit bacterial growth and/or eradicate the infection.

The invention is a further development of an antimicrobial peptide similar to the sequence of the "bovine lactoferricin" peptide Com2. The improved physicochemical properties and increased antimicrobial activity of the peptide P14 are due to the introduction of novel, unnatural amino acids (Hdn as defined above). This amino acid combines hydrophobic as well as ionic properties in one amino acid and thus increases the amphiphilic character of the peptide. This results in a structural stabilisation of the amphiphilic peptide secondary structure by hydrophobic side chain interaction of the amino acid and increases the antimicrobial activity as well as proteolytic stability in serum.

Parental peptide sequence (left) and further development P14 (invention, right). The modifications are marked and include the introduction of 4-acetamidobenzoic acid at the N-terminus and replacement of tryptophan with the unnatural amino acid dinaphtylhistidine (Hdn).

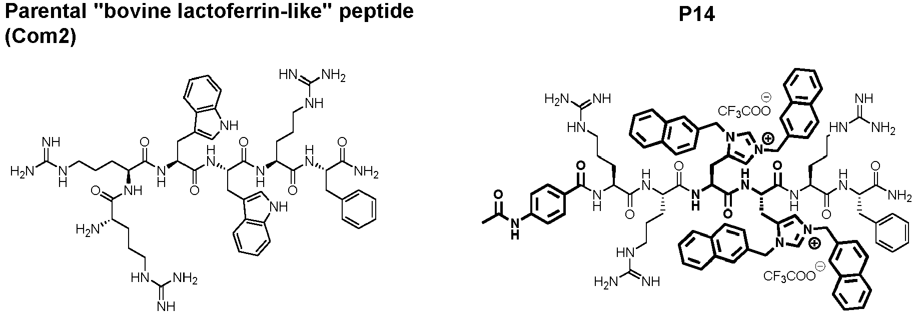

The synthesis of the unnatural amino acid Hdn used in the P14 peptide can involve three reaction steps starting from commercially available Fmoc-protected histidine.

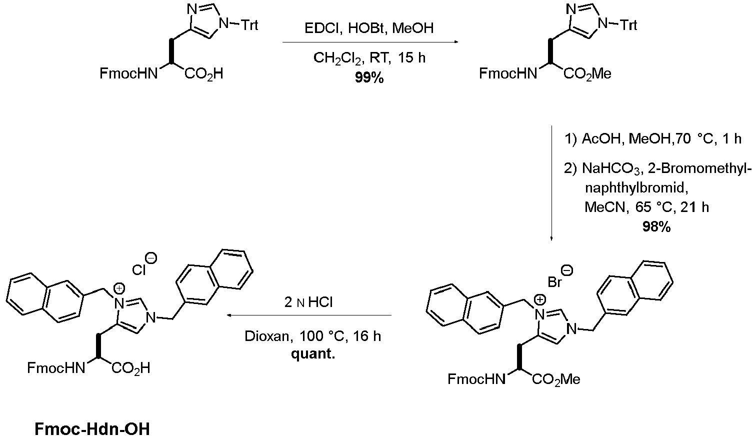

The main advantages of the P14 peptide are: 1) The simple and cost-effective synthesis via linear solid-phase peptide synthesis compared to the recombinant synthesis of conventional antimicrobial peptides. The unnatural amino acid incorporated here can be easily synthesised in three steps starting from commercially available Fmoc-protected histidine. 2) The peptide P14 has a low molecular mass and thus reduces the probability of immune reactions 3) The peptide P14 shows antimicrobial activity against multi-resistant bacterial strains (ESKAPE; E.cloacae, S.aureus, K.pneumoniae, A.baumannii, P.aeruginosa, E.faecium). 4) In model organisms for Gram-negative (E. coli) and Gram-positive (B. subtilis), increased selectivity towards Gram-positive strains was observed. 5) The peptide P14 has an increased proteolytic stability in human serum, which increases the metabolic half-life and improves treatment of infections in vivo. The minimum inhibitory concentration is well below the haemolytic and cytotoxic concentration and thus offers a distinct concentration window for the treatment of infections. 6) The peptide P14 has been shown to have a more specific antibacterial mechanism of action than lysis of the bacterial membrane. Inhibition of peptidoglycan synthesis (membrane system of the bacterium) was identified as one possible mechanism of action, without being bound to any theory.

In the following, the invention is explained in more detail with reference to the examples and figures. It is expressly pointed out that the examples and figures are not to be understood as limiting the invention thereto.

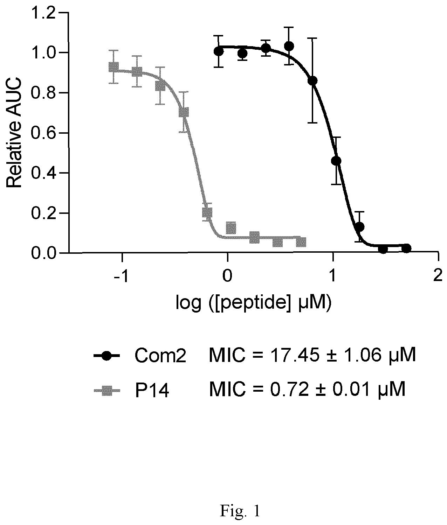

Figure 1 shows the minimum inhibitory concentration (MIC) of parent Com2 and peptide P14 against *Bacillus subtilis.* The experiments were performed in Mueller-Hinton medium in three independent experiments.

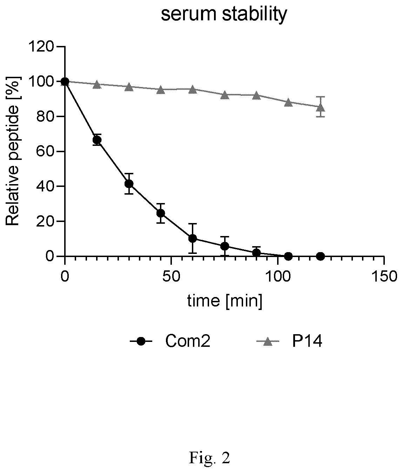

Figure 2 shows the serum stability of the peptides Com2 and P14. The experiment was performed in RPMI medium with 30% human serum at 37°C. Aliquots of the solution were analysed by HPLC-MS and the relative content was determined using an internal standard (phenol). The experiment was performed in three independent experiments.

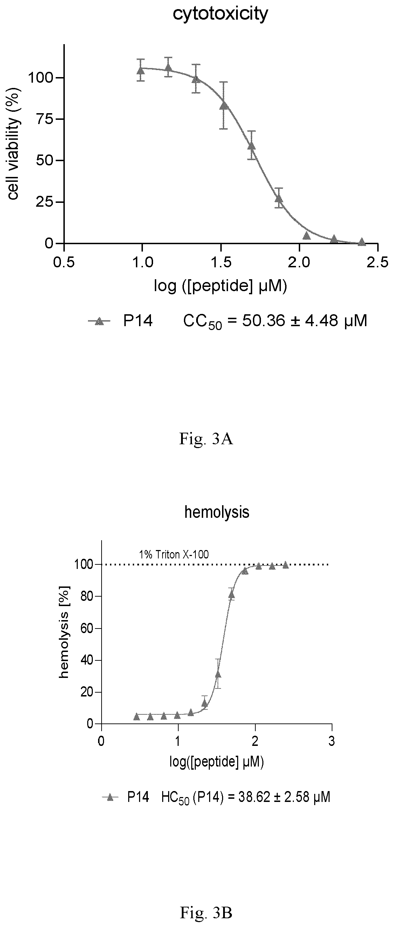

Figures 3A and 3B show the viability of HCT116 cells in DMEM with 10% FBS and 1% penicillin/streptomycin (Fig. 3A). The haemolysis experiments were performed with fresh blood from a healthy donor. Both experiments were performed in three independent experiments.

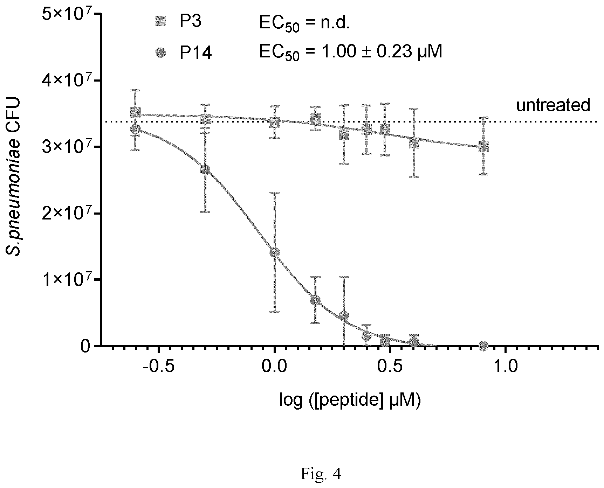

Figure 4 shows the dose-dependent treatment of infected A549 lung cells with P3 (control, Aba-RRWWRF-CONH₂, SEQ ID. No. 1) or P14 peptide. The number of surviving bacterial colonies after treatment with peptide is shown.

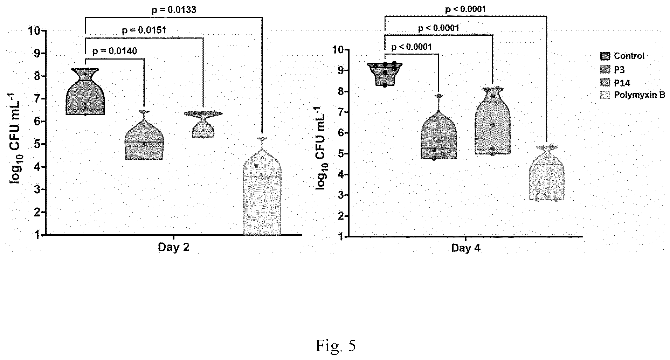

Fig. 5 shows a mouse model for the treatment of skin infection with P3 (control) or P14 peptide against *A*. *baumannii.* The number of surviving bacterial colonies after treatment with peptide is shown.

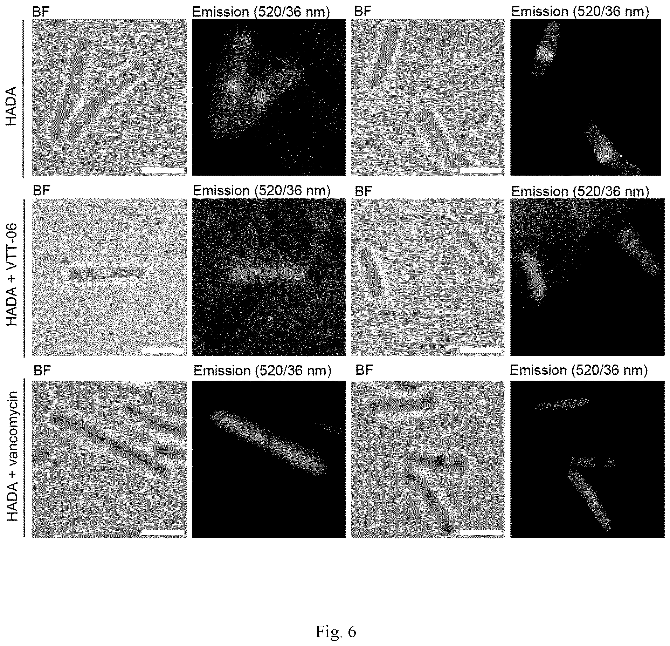

Fig. 6 shows the fluorescence microscopy of B. subtilis NCIB 3610, stained with HADA. Scale bars represent 2 µm. BF are the bright light images.
Fig. 7 shows the antimicrobial activity of the peptides P15, P16, and P17.

### EXAMPLES

In the following, the synthesis of a compound according to the present invention is described.

### Synthesis of Fmoc-L-His(Trt)-OMe (2)

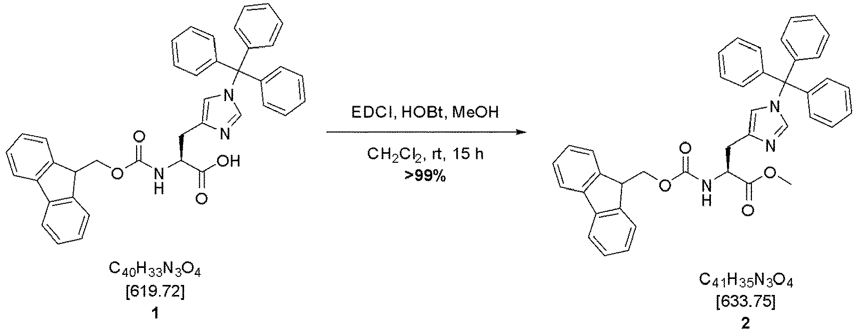

Following a procedure of Bang *et al*.,^{[4]} Fmoc-L-His(Trt)-OH (15.0 g, 24.2 mmol, 1.00 equiv) was suspended in CH₂Cl₂ (100 mL). To this solution EDC hydrochloride (5.10 g, 26.6 mmol, 1.10 equiv) and HOBt-H₂O (3.60 g, 26.6 mmol, 1.10 equiv) were at rt. After complete dissolution, MeOH (20 mL, 488 mmol, 20.1 equiv) was added and stirred for 15 h. The mixture was diluted with CH₂Cl₂ and washed with H₂O. The organic layer was dried over MgSO₄, filtered and concentrated. The residue was purified by silica column chromatography using CH₂Cl₂/MeOH 50:1 as eluent to yield Fmoc-L-His(Trt)-OMe (15.3 g, 24.2 mmol, > 99%) as a white foam.

**TLC:** *R_{f}* = 0.36 (CH₂Cl₂/MeOH 50:1).

**¹H-NMR:** 500 MHz, 300 *K,* DMSO-*d*₆; *δ* (ppm) = 7.89 (d, ³*J* = 7.6 Hz, 2H, CH_{ar,Fmoc}), 7_{·}74 (d, ³*J* = 8.0 Hz, 1H, NH), 7.67 (d, ³*J* = 7.5 Hz, 2H, CH_{ar,Fmoc}), 7.43-7.26 (m, 14H, ε¹-CH, 9x Trt, 4x CH_{ar,Fmoc}), 7.05-7.03 (m, 6H, CH_{ar,Trt}), 6.69 (s, 1H, δ²-CH), 4.33-4.30 (m, 1H, α-CH), 4.28-4.26 (m, 1H, C*H*H_{Fmoc}) , 4.24-4.21 (m, 1H, CH*H*_{Fmoc}), 4.17 (t, ³*J* = 7.0 Hz, 1H, CH_{Fmoc}), 3.57 (s, 3H, OMe), 2.89 (dd, ²*J* = 14.4 Hz, ³*J* = 5.5 Hz, 1H, β-C*H*H), 2.81 (dd, ²*J* = 14.4 Hz, ³*J* = 8.6 Hz, 1H, β-CHH) .

**¹³C-NMR:** 125 MHz, 300 *K,* DMSO-*d*₆; *δ* (ppm) = 172.4 (CO), 155.9 (CO_{Fmoc}), 143.8 (C_{q,ar,Fmoc}), 143.7 (C_{q,ar,Fmoc}), 142.2 (C_{q,ar,Trt}), 140.74 (C_{q,ar,Fmoc}), 140.72 (C_{q,ar,Fmoc}), 137.9 (ε¹-CH), 136.4 (γ-C), 129.2 (CH_{ar,Trt}), 128.2 (CH_{ar,Trt}), 128.0 (CH_{ar,Trt}), 127.7 (C_{ar,Fmoc}), 127.1 (C_{ar,Fmoc}), 125.2 (C_{ar,Fmoc}), 120.2 (C_{ar,Fmoc}), 119.2 (δ²-CH), 74.5 (*C*Ph₃), 65.7 (CH_{2, Fmoc}), 54.2 (α-CH), 51.9 (OMe), 46.6 (CH_{Fmoc}), 29.9 (β-CH₂).

**HRMS (ESI⁺):** calcd for C₄₁H₃₅N₃O₄H⁺ [M+1H]⁺¹: 634.2700, found.: 634.2697.

### Synthesis of Fmoc-L-Hdn-OMe (3)

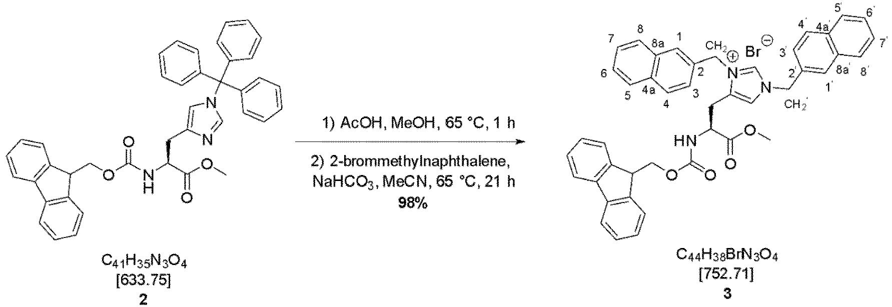

Fmoc-L-His(Trt)-OMe (3.17 g, 5.00 mmol, 1.00 equiv) was suspended in MeOH/AcOH (1:1, 40 mL) and refluxed for 1 h. After deprotection completion, the mixture was concentrated and co-evaporated with toluene to remove AcOH. The residue was dissolved in MeCN (40.0 mL) and NaHCO₃ (0.80 g, 10.0 mmol, 2.00 equiv) and 2-(bromomethyl) naphthalene (5.53 g, 25.0 mmol, 5.00 equiv) were added. The mixture was stirred at 65 °C for 21 h. After cooling to room temperature, the solvent was evaporated under reduced pressure. The residue was suspended in CH₂Cl₂, filtered and concentrated. The residue was purified by silica column chromatography using CH₂Cl₂/MeOH 10:1 to yield Fmoc-L-Hdn-OMe bromide (3.70 g, 4.92 mmol, 98%) as an off-white foam.

**TLC:** R*_{f}* = 0.27 (CH₂Cl₂/MeOH 10:1).

**¹H-NMR:** 500 MHz, 300 *K,* DMSO-*d*₆; *δ* (ppm) = 9.39 (d, ⁴*J* = 1.0 Hz, 1H, ε¹-CH) , 7.99 (d, ³*J* = 8.5 Hz, 1H, 4-CH), 7.96-7.94 (m, 2H, NH, CH_{ar,Naph}), 7.91-7.88 (m, 7H, 1'-CH, , 2x CH_{ar,Fmoc}, 4x CH_{ar,Naph}), 7.84 (s, 1H, 1-CH), 7.68 (s, 1H, δ²-CH), 7.62-7.51 (m, 6H, 2x CH_{ar,Fmoc}, 4× CH_{ar,Naph}), 7.48-7.44 (m, 2H, 3-CH, 3'-CH), 7.41 (dt, ³*J* = 7.4 Hz, ⁴*J* = 2.7 Hz, 2H, CH_{ar,Fmoc}), 7.29 (t, ³*J* = 7.4 Hz, 2H, CH_{ar,Fmoc}), 5.64 (s, 2H, CH₂), 5.57 (s, 2H, CH₂'), 4.38-4.34 (m, 1H, α-CH), 4.28 (dd, ²*J* = 9.1 Hz, ³*J* = 5.5 Hz, 1H, C*H*H_{Fmoc}), 4.14-4.08 (m, 2H, CH_{Fmoc}, C*H*H_{Fmoc}), 3.57 (s, 3H, OMe), 3.17 (dd, ²*J* = 16.0 Hz, ³*J* = 4.6 Hz, 1H, β-C*H*H), 3.00 (dd, ²*J* = 16.0 Hz, ³*J* = 9.7 Hz, 1H, β-CH*H*).

**¹³C-NMR:** 125 MHz, 300 *K,* DMSO-*d*₆; *δ* (ppm) = 170.8 (CO), 155.8 (CO_{Fmoc}), 143.61 (C_{q,ar,Fmoc}), 143.57 (C_{q,ar,Fmoc}), 140.7 (C_{q,ar,Fmoc}), 136.9 (ε¹-CH), 132.73 (C_{q,ar,Naph}), 132.70 (C_{q,ar,Naph}), 132.66 (C_{q,ar,Naph}), 132.0 (C_{q,ar,Naph'}), 131.6 (γ-CH), 131.3 (C_{q,ar,Naph}), 128.9 (4-CH), 128.7 (4'-CH), 127.8 (CH_{ar,Naph}), 127.8 (CH_{ar,Naph}), 127.7 (CH_{ar,Naph}), 127.6 (CH_{ar,Fmoc}), 127.4 (C_{q,ar,Naph'}), 127.03 (1-CH), 127.00 (CH_{ar,Fmoc}), 126.74 (C_{Naph'}), 126.70 (CH_{ar,Naph'}), 125.5 (3'-CH), 125.2 (3-CH), 125.1 (CH_{ar,Fmoc}), 125.0 (CH_{ar,Fmoc}), 121.0 (δ²-CH), 120.1 (CH_{ar,Fmoc}), 65.7 (CH_{2,Fmoc}), 52.2 (OMe), 52.1 (α-CH), 49.9 (CH₂), 46.5 (CH_{Fmoc}), 25.1 (β-CH₂).

HRMS **(ESI⁺)**: calcd for C₄₄H₃₈N₃O₄⁺ [M+1H]⁺¹: 672.2857, found: 672.2854.

### Synthesis of Fmoc-L-Hdn-OH (4)

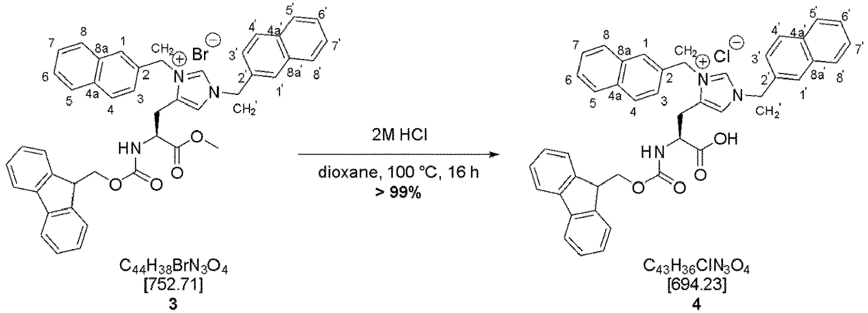

To a solution of Fmoc-L-Hdn-OMe bromide (1.50 g, 1.98 mmol, 1.00 equiv) in 1,4-dioxane (5.50 mL) was added 2 M aq. HCl (5.50 mL, 5.50 equiv). The mixture was stirred at 100 °C for 16 h. After cooling to rt, the solvent was evaporated under reduced pressure to yield Fmoc-L-Hdn-OH chloride (1.37 g, 1.97 mmol, >99%) as a pale-yellow foam.

**TLC:** R*_{f}* =0.41 (CH₂Cl₂/MeOH/AcOH 10:1:0.01.)

**¹H-NMR:** 500 MHz, 300 *K,* DMSO-*d*₆; *δ* (ppm) = 13.00 (brs, 1H, CO₂H), 9.46 (d, ⁴*J* = 0.9 Hz, 1H, ε¹-CH), 7.99 (d, ³*J* = 8.5 Hz, 1H, CH_{ar,Naph}), 7.96-7.94 (m, 1H, CH_{ar,Naph}), 7.89-7.86 (m, 7H, NH, 1'-CH, CH_{ar,Fmoc}, CH_{ar,Naph}), 7.85 (s, 1H, 1-CH), 7.69 (s, 1H, δ²-CH), 7.63-7.58 (m, 2H, CH_{ar,Fmoc}), 7.57-7.55 (m, 2H, CH_{ar,Naph}), 7.52-7.50 (m, 2H, CH_{ar,Naph}), 7.48 (m, 1H, 3'-CH), 7.46 (m, 1H, 3-CH), 7.40 (dt, ³*J* = 7.4 Hz, ⁴*J* = 3.4 Hz, 2H, CH_{ar,Fmoc}), 7.28 (t, ³*J* = 7.4 Hz, CH_{ar,Fmoc}), 5.66 (d, ⁴*J* = 1.6 Hz, 2H, CH₂), 5.59 (s, 2H, CH₂'), 4.34-4.30 (m, 1H, α-CH), 4.23 (dd, ²*J* = 10.0 Hz, ³*J* = 6.7 Hz, C*H*H_{Fmoc}), 4.12 (t, ³*J* = 6.8 Hz, 1H, CH_{Fmoc}), 4.06 (dd, ²*J* = 10.0, ³*J* = 7.2 Hz, 1H, C*H*H_{Fmoc}) , 3.18 (dd, ²*J* = 16.0 Hz, ³*J* = 4.1 Hz, 1H, β-C*H*H), 3.00 (dd, ²*J* = 16.0 Hz, ³*J* = 10.1 Hz, 1H, β-CHH) .

**¹³C-NMR:** 125 MHz, 300 *K,* DMSO-*d*₆; *δ* (ppm) = 171.8 (CO), 155.9 (CO_{Fmoc}), 143.65 (C_{q,Fmoc}), 143.60 (C_{q,Fmoc}), 140.7 (C_{q,Fmoc}), 136.8 (ε¹-CH), 132.7 (C_{q,Naph}), 132.7 (C_{q,Naph}), 132.7 (C_{q,Naph}), 132.1 (C_{q,Naph'}), 132.0 (γ-C), 131.4 (C_{q,Naph}), 128.9 (CH_{ar,Naph}), 128.7 (C_{q,Naph}), 127.9 (CHₐᵣ), 127.8 (CHₐᵣ), 127.7 (CH_{ar,Naph}), 127.6 (CH_{ar,Fmoc}), 127.6 (CH_{ar,Fmoc}), 127.4 (CH_{ar,Naph}), 127.03 (CH_{ar,Fmoc}), 127.01 (1-CH), 126.7 (CH_{ar,Naph}), 126.7 (CH_{ar,Naph}), 125.5 (3-CH), 125.2 (3-CH), 125.2 (CH_{ar,Fmoc}), 125.1 (CH_{ar,Fmoc}), 120.8 (δ²-CH), 120.1 (CH_{ar,Fmoc}), 65.7 (CH_{2,Fmoc}), 52.2 (CH₂'), 52.1 (α-CH), 49.9 (CH₂), 46.5 (CH_{Fmoc}), 25.3 (β-CH₂).

**HRMS (ESI⁺)**: calcd for C₄₃H₃₆N₃O₄⁺ [M+1H]⁺¹: 658.2700, found: 658.2731.

### 1. Antimicrobial activity of the P14 peptide

The peptide P14 demonstrates an increased antimicrobial effect against Bacillus Subtilis due to the introduced Hdn amino acid. This is evident from the > 24-fold higher minimum inhibitory concentration (MIC) compared to the parent peptide (Com2).

The peptide was also tested against multi-resistant ESKAPE pathogens and shows MIC values in the micromolar range. Especially against Gram-positive strains a high potency is noted.

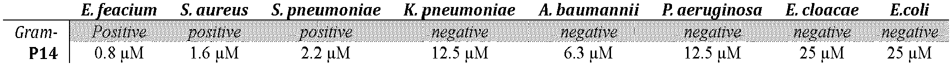

Table 1: The minimum inhibitory concentrations (MIC) of peptide P14 against different ESKAPE pathogens. The experiments were performed in Mueller-Hinton medium in three independent experiments.

### 2. Proteolytic serum stability

The P14 peptide shows a significantly increased stability (Com2, t1/2 = ~ 24 min; P14, t1/2 = > 14 h) in human serum compared to the parent peptide Com2 and thus indicates compatibility for subcutaneous administration, but an oral administration is also envisaged.

### 3. Cell and haemolysis experiments

Treatment of eukaryotic HCT116 cells shows a cytotoxic concentration of CC50 = 50.36 ± 4.48 µM. This is therefore > 65-fold higher than the minimum inhibitory concentration against B. subtilis. This also applies to the haemolytic concentration, which is HC50 = 38.62 ± 2.58 µM and is > 50-fold higher than the MIC value. Consequently, the P14 peptide as an antibiotic offers a distinct concentration window for therapeutic use.

### 4. Infection model 1 - treatment of K. pneumoniae-infected lung cells

The peptide P14 was tested as an antibiotic against the treatment of K. pneumoniae-infected A549 lung cells. Compared to the P3, which was used as a control peptide and already carries the N-terminal modification, P14 showed a complete inhibition of bacterial growth at 8 µM.

### 5. Infection model 2 - treatment of A. baumanii infected mice

Infection of the mice was induced by superficial abrasion and incubation with the bacterial culture. As in infection model 1, the P14 peptide shows a higher activity than the control peptide P3. Furthermore, the potency of the antimicrobial peptide can be compared with that of the commonly used polymyxin B.

### 6. Inhibition of membrane synthesis

Fluorescence microscopy of B. subtilis stained with HADA, showing newly synthesised peptidoglycan from control cells. By treatment with vancomycin and the peptide, no HADA binding to the cell periphery is visible. The peptide P14 thus inhibits bacterial polyglycan synthesis in a manner comparable to conventional vancomycin.

### 7.Further compounds according to the present invention and their inhibitory effect

The amino acids Hdm, Hdt and Hdb have been synthesized analogously to the Hdn amino acid. Most interestingly Hdt and Hdb follow the same principle of cationic histidinium charge and hydrophobic substituents that were introduced through the alkylation of N1 and N3.

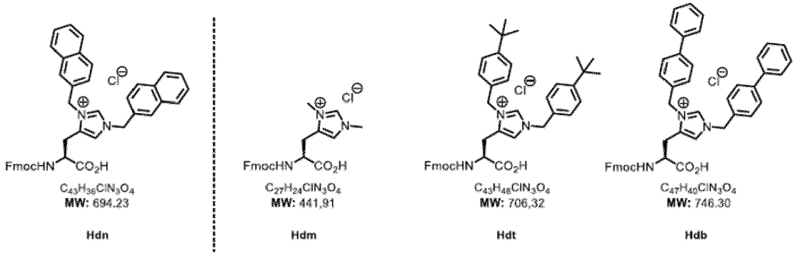

Analogously to P14, these amino acids have been incorporated into the parental sequence of Com2 and were partially tested on their antimicrobial activity against Gram-positive *Bacillus Subtilis* 3610 NCIB.

| **Peptide** | **Sequence** |
|---|---|
| P15 | Aba-RR-Hdm-Hdm-RF-CONH₂ (SEQ ID no.17) |
| P16 | Aba-RR-Hdt-Hdt-RF-CONH₂ (SEQ ID no.17) |
| P17 | Aba-RR-Hdb-Hdb-RF-CONH₂ (SEQ ID no.17) |

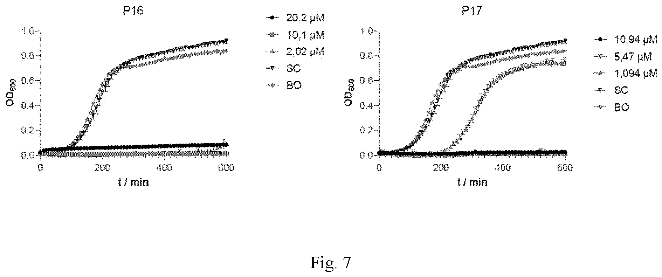

These peptides have displayed antimicrobial activity in the low µM rang (see Figure 7).

In summary, the invention was tested in the following experiments (the detailed results can be found above): 1) Antimicrobial activity against Gram-positive (*B. subtilis*) and -negative (*E.coli*) as well as multidrug-resistant strains (ESKAPE) was tested. The peptide P14 is active against a large number of strains and inhibits bacterial growth. 2) Serum stability was tested using human serum and shows a significantly higher stability of peptide P14 compared to the parent peptide Com2. 3) Haemolysis experiments using human blood from a healthy donor show a haemolytic concentration >50-fold above the minimum inhibitory concentration against B. subtilis. 4) Fluorescence microscopy experiments demonstrated a specific mechanism of action of peptide P14, which is based on the inhibition of peptidoglycan synthesis and not on the lysis of the bacterial cell membrane. 5) Cell experiments demonstrate a cytotoxic concentration > 65-fold above the minimum inhibitory concentration against *B. subtilis. 6*) Infection models based on cell experiments (K. pneumoniae-infected A549 lung cells) and mouse experiments (superficial *A. baumannii* infection and mice) showed successful treatment of the respective infection.

## Claims

1. Compound for substituting amino acids of proteins or peptides, wherein the compound has the formula (1) 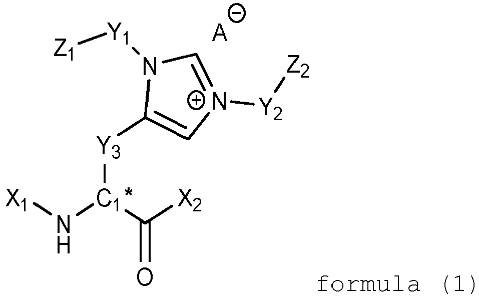 wherein
the stereocenter C₁* represents C*H* that either results in the D- or L-form of the compound of formula (1);
A⁻ is a negatively charged ion compensating the positive charge;
X₁ is H or an amine-protecting group;
X₂ is OH, an ester residue, an amide residue, or an activated carboxylic acid derivate to be used for the formation of an amide bond;
Y₁, Y₂ and Y₃ are independently from each other a C₁-C₆ alkyl, and
Z₁ and Z₂ are independently from each other a nucleobase, a C1 to C16 alkyl group, an aromatic residue or a heteroaromatic residue, wherein the aromatic residue or the heteroaromatic residue contains one 5, 6, or 7-membered ring or two condensed 5, 6, or 7-membered rings sharing two carbon atoms, wherein the aromatic residue or the heteroaromatic residue can be substituted by a linear or branched C1 to C16 alkyl group and/or a further aromatic group.

2. The compound according to claim 1, wherein A⁻ is a conjugate base selected from the group consisting of formic acid, acetic acid, trifluoroacetic acid or halogen anions.

3. The compound according to any of the preceding claims, wherein the amine-protecting group is selected from the group consisting of tert-butyloxycarbonyl, allyloxycarbonyl, and the fluorenyl methoxycarbonyl protecting group.

4. The compound according to any of the preceding claims, wherein the activated carboxylic acid derivate is selected from the group consisting of halogenides, N-hydroxysuccinimide ester, hydroxybenzotriazole ester and p-nitrophenyl ester.

5. The compound according to any of the preceding claims, wherein the C₁-C₆ alkyl is CH₂.

6. The compound according to any of the preceding claims, wherein Y₁ = Y₂ = Y₃, Y₁ ≠ Y₂ = Y₃, Y₁ = Y₂ ≠ Y₃, Y₁ = Y₃ ≠ Y₂ or Y₁ ≠ Y₂ ≠ Y₃.

7. The compound according to any of the preceding claims, wherein Z₁ = Z₂.

8. The compound of formula (1), wherein Z₁ and Z₂ are naphthyl.

9. Method for preparing the compound of formula (1) according to any of claims 1 to 8, comprising the following steps:
esterification of Fmoc-His(Trt)-OH;
proton-catalyzed trityl (Trt) group deprotection;
direct dialkylation of the imidazole moiety, and
hydrolysis of the ester.

10. Protein or peptide, in which at least one amino acid is substituted by the compound of formula (1) according to any of claims 1 to 8, wherein the peptide is preferably an antimicrobial peptide, in particular an antimicrobial peptide having the formula H₂N-RRWWRF-CONH₂.

11. Protein or peptide according to claim 10, wherein one to six amino acids of the protein or peptide are replaced by the compound of formula (1).

12. Protein or peptide according to any of claims 10 to 11, wherein the N-terminus of the protein or peptide, in which at least one amino acid is substituted by the compound of formula (1) is modified.

13. Protein or peptide according to any of claims 10 to 12 having the formula RR-XX-RF-CONH₂, wherein X is the compound of formula (1), optionally substituted at the N-terminus by Aba, wherein Aba represents 4-acetamido benzoic acid residue.

14. Use of the compound of formula (1) as defined in any of claims 1 to 8 for replacing at least one amino acid of a protein or a peptide.

15. Protein or peptide as defined in any of claims 10 to 13 for the therapy of infections.
